# EUROPÄISCHE PATENTANMELDUNG

(11) **EP 1 762 257 A1**
(43) Veröffentlichungstag der Anmeldung: **14.03.2007**
(21) Anmeldenummer: 06016688.1
(22) Anmeldetag: 10.08.2006
(51) Int. Cl.: A61M 1/36, F16K 7/10, F16K 7/07, A61M 39/22

(54) **Vorrichtung zur Unterbrechung eines durch einen Hohlkörper fliessenden Blutstroms in einem extrakorporalen Blutkreislauf**

(30) Priorität: 13.09.2005 DE 102005045663
(71) Anmelder: Novalung GmbH, 72379 Hechingen (DE)
(72) Erfinder: Spranger, Martin, 72119 Entringen (DE)
(74) Vertreter: Laufer, Gabriele

(57) **Zusammenfassung**

Die vorliegende Erfindung betrifft eine Vorrichtung (10) zur Unterbrechung eines durch einen Hohlkörper (12) fließenden Blutstroms, wobei die Vorrichtung einen röhrenförmigen Hohlkörper und ein in dem Hohlkörper angeordnetes dilatierbares Element (14) mit einer Öffnung aufweist. Über die Öffnung steht das zumindest eine dilatierbare Element mit einem Fluidanschluss in Fluidverbindung, wobei das zumindest eine dilatierbare Element durch Zuleitung eines Fluids aus dem Fluidanschluss durch die Öffnung von einem nicht-dilatierten in einen dilatierten Zustand überführbar ist. Ferner sind Mittel (22) im Fluidanschluss vorgesehen, welche die Zuleitung des Fluids in das zumindest eine dilafierbare Element steuern, wobei die Mittel über ein zweites Fluid steuerbar sind.

## Beschreibung

Die vorliegende Erfindung betrifft eine Vorrichtung zur Unterbrechung eines durch einen Hohlkörper fließenden Blutstroms in einem extrakorporalen Kreislauf, mit einem dilatierbaren Element, welches von einem nicht-dilatierten Zustand in einen dilatierten Zustand überführt werden kann, und welches im dilatierten Zustand den Blutstrom unterbricht.

Extrakorporale blutführende Systeme werden bspw. im Rahmen einer extrakorporalen Lungenunterstützung eingesetzt, um Patienten bei gestörter Lungenfunktion extrakorporalen Gasaustuasch zu bieten.

Bei extrakorporalen Kreisläufen und insbesondere unter Zwischenschaltung von Geräten wie Oxygenatoren oder Dialysatoren besteht oftmals die Gefahr, dass Gas in das Blutkreislaufsystem eindringt und über das Schlauchsystem in die Blutgefäße des Patienten gelangt, und dort Embolien auslöst. Um dies zu vermeiden, ist eine schnelle Unterbrechung des Blutstromes notwendig. Dabei werden die blutführenden Schläuche üblicherweise abgeklemmt, sobald erkannt wird, dass Gase in Form von Blasen um Blutstrom mitgeführt werden.

Herkömmliche Techniken umfassen manuell oder automatisch betätigte Vorrichtungen, mit welchen der Blutfluss durch die Schläuche mittels Druck gegen die äußeren Schlauchwände unterbrochen werden kann.

Nachteilig an diesen herkömmlichen Vorrichtungen ist in erster Linie - bedingt durch den benötigt hohen Kraftaufwand - die Verzögerungszeit, was den Einsatz solcher Systeme bei den immer kürzer werdenden externen Verweilzeiten des Blutes verbietet.

Aus dem europäischen Patent EP 0 373 847 ist ein Membranoxygenator bekannt, bei welchem eine Einrichtung zur automatischen Aufrechterhaltung des Druckes vorgesehen ist. Die Einrichtung weist unter anderem ein Ventil mit einem inneren Schlauch auf, auf welchen von außen Druck ausgeübt werden kann. Das Ventil dient darüber hinaus dazu, die Strömung des aus dem Oxygenator's austretenden Sauerstoffanreicherungsgases durch den Druck des aus dem Oxgenator austretenden Blutes zu drosseln. Das hier offenbarte Ventil dient also lediglich der Aufrechterhaltung eines Druckes, mit welchem das Sauerstoffanreicherungsgas im Oxygenator reguliert wird.

So offenbart bspw. die DE 40 12 525 eine Betätigungsvorrichtung für ein Schlauchsystem, wobei mit der Betätigungsvorrichtung wahlweise Schlauchquerschnitte blockiert oder freigegeben werden können. Auch bei dieser Vorrichtung wird jedoch der Schlauch, bzw. die Schlauchabschnitte mechanisch zugedrückt, sollte eine Blockierung des Schlauchsystems gewünscht werden.

Aus der EP 0 706 343 B1 ist ein Kreislaufsystem mit einer Blutpumpe und einer Okklusionsvorrichtung bekannt, wobei die Blutpumpe über eine Leitung in Strömungsverbindung mit dem Blutgefäß der zu behandelnden Person steht. Ferner ist eine Verschlussvorrichtung zur Strömungsregelung der Blutpumpe vorgesehen. Außerdem umfasst das System Regelungsmittel für die Inbetriebnahme der Verschlussvorrichtung bei einer Fehlfunktion der Blutpumpe.

Aufgabe der vorliegenden Erfindung ist es daher, eine Vorrichtung bereitzustellen, mit welcher ein Fluidstrom, insbesondere in extrakorporalen Blutkreisläufen, sehr schnell und effektiv unterbrochen werden kann.

Diese Aufgabe wird erfindungsgemäß durch eine Weiterbildung der eingangs genannten Vorrichtung gelöst, bei welcher die Vorrichtung ferner zumindest ein in dem blutführenden Hohlkörper angeordnetes dilatierbares Element mit einer Öffnung aufweist, über welche das zumindest eine dilatierbare Element mit einem mit einem Fluidanschluss in Fluidverbindung steht, wobei das zumindest eine dilatierbare Element durch Zuleiten eines Fluids aus dem Fluidanschluss durch die Öffnung von einem nicht-dilatierten in einen dilatierten Zustand überführbar ist, wobei ferner Mittel im Fluidanschluss vorgesehen sind, welche die Zuleitung des Fluids in das zumindest eine dilatierbare Element steuern, wobei die Mittel über ein zweites Fluid steuerbar sind.

Ferner betrifft die Erfindung ein Verfahren zur Unterbrechung eines Blutstromes in einem extrakorporalen Kreislauf, wobei die erfindungsgemäße Vorrichtung eingesetzt wird.

Schließlich besteht die erfindungsgemäße Verwendung in der Anwendung der erfindungsgemäßen Vorrichtung zur Unterbrechung eines Blutstroms in einem extrakorporalen Kreislauf.

Die der Erfindung zu Grunde liegende Aufgabe wird dadurch vollkommen gelöst. Mit der erfindungsgemäßen Vorrichtung ist es zum ersten Mal möglich, einen Fluidstrom in einem Hohlkörpersystem, bspw. der Blutstrom in einem Blutschlauchsystem, effektiv und sehr schnell zu unterbrechen. Die Aktivierung des Systems erfolgt bspw. mittels eines vorgeschalteten Blasen-Detektors, welcher gegebenenfalls die Vorrichtung akitiviert.

Die Erfindung eignet sich insbesondere bei Schlauchsystemen von extrakorporalen Blutkreisläufen, bei welchen es notwendig ist, den Blutstrom schnell zu unterbrechen, falls gasförmige oder korpuskuläre Stoffe in dem in den menschlichen Körper zurückzuführenden Blut erkannt werden. Die erfindungsgemäße Vorrichtung ist jedoch nicht auf extrakorporale Blutkreisläufe beschränkt, sondern kann auch zu jedem anderen Zeck eingesetzt werden, bei welchem es notwendig ist, den Fluss eines Fluids mittels eines einfachen und schnellen Vorgangs effektiv zu unterbinden.

Hierbei ist bevorzugt, wenn der Fluidanschluss ein Reservoir ist.

Bevorzugt ist ferner, wenn das Reservoir außerhalb des Hohlkörpers angeordnet ist. Durch das außerhalb des Hohlkörpers angeordnete Reservoir kann das Fluid aus dem Reservoir über eine Öffnung in das dilatierbare Element geleitet werden, wodurch das dilatierbare Element in einen expandierten Zustand überführt wird und dadurch den Strömungsweg blockiert. Das Fluid, mit welchem das dilatierbare Element in den dilatierten Zustand überführt wird kann entweder eine Flüssigkeit oder ein Gas geeigenter Viskosität sein.

Mit den im Reservoir vorgesehenen Mitteln kann die Zuleitung des Fluids, mit welchem das dilatierbare Element in einen dilatierten Zustand überführbar ist, gesteuert werden. Durch die Steuerung des Fluids gelangt dieses aus dem Reservoir über die Öffnung in das dilatierbare Element, das sich dadurch erweitert und den Hohlkörper verschließt.

Dabei ist insbesondere bevorzugt, wenn die Volumenmenge an Fluid, die in dem Reservoir enthalten ist, zumindest der Volumenmenge des dilatierbaren Elements im expandierten Zustand entspricht.

Dadurch wird gewährleistet, dass das dilatierbare Element genügend expandiert wird, um den Hohlkörper vollständig zu verschließen. Der röhrenförmige Hohlkörper kann dabei entweder ein Schlauch, ein Konnektor oder ein sonstiges röhrenförmiges System sein.

In einer Ausführungsform ist bevorzugt, wenn das zumindest eine dilatierbare Element ein Ballon ist. Der Ballon kommt dann im nicht-expandierten Zustand in der Strömung des durch das Hohlkörpersystem fließenden Fluids zu liegen, und verschließt durch seine Überführung in den dilatierten Zustand diesen Strömungsweg.

Dabei ist insbesondere bevorzugt, wenn der Ballon im expandierten Zustand an eine ringförmige Verengung im Strömkanal abdichtend anliegt. Dadurch wird gewährleistet, dass auch bei hohem Strömdruck der Fluidfluss effektiv und vollständig unterbrochen wird. Der expandierte Ballon legt sich dabei mit seiner äußeren Hülle an die seiner äußeren Formgebung angepasste ringförmige Verengung an, wobei der anliegende Strömungsdruck die Dichtfunktion unterstützt.

In einer anderen Ausführungsform kann bspw. das dilatierbare Element ein dilatierbarer innerer Schlauch sein, der - nach Art eines Ringes - im Querschnitt des Hohlkörpers umlaufend an dessen Innenwand angebracht ist und sich bei Zuleitung eines Fluids durch die Öffnung in den inneren Schlauch derart expandieren lässt, dass das Hohlkörpersystem durch den voll expandierten inneren Schlauch verschlossen und der durch das Hohlkörpersystem fließende Blutstrom abgeschnürt wird.

Bei dieser Ausführungsform erfolgt danach die Unterbrechung des Hohlkörpersystems und die Unterbrechung des Blutstromes von - mit Bezug auf den Querschnitt des Hohlkörpersystems - außen nach innen. Bei dem zuvor erwähnten Ausführungsbeispiel des dilatierbaren Ballons, der im Hohlkörper angeordnet ist, findet die Blockierung des Hohlkörpers, bzw. des durch diesen fließenden Stroms, von innen nach außen statt.

Auch durch die zuletzt genannte Ausführungsform wird gewährleistet, dass ein schneller Verschluss des Hohlkörpersystems vorgenommen werden kann, sollte dies bspw. im Falle von Eindringen von Gasen in das Blut gewünscht werden.

Es ist vom technischen Standpunkt gesehen klar, dass die Anordnung solcher expandierbarer Elemente in einer Vielzahl von Anordnungen denkbar ist.

In einer bevorzugten Ausführungsform stellen die im Reservoir vorgesehenen Mittel einen Kolben dar, der über ein zweites Fluid verschiebbar im Reservoir angebracht ist.

Bei dieser Ausführungsform kann somit - wenn ein in einem Hohlkörper strömendes Fluid unterbrochen werden soll - über ein zweites Fluid ein Kolben in das Reservoir eingeführt werden, wodurch das in dem Reservoir enthaltene Fluid durch Verdrängung aus dem Reservoir über die Öffnung des dilatierbaren Elements in eben dieses gepresst wird. Diese Ausführungsform hat den Vorteil, dass eine exakt dosierte Menge Fluid schnell in das expandierende Element überführt werden kann und hier einen genau definierbaren Druck erzeugt.

Es versteht sich, dass der Kolben abdichtend im Reservoir geführt ist, d.h. dass der Kolben zusammen mit dem Reservoir den Raum für das erste Fluid begrenzt.

Die Kolbenbewegung wird dabei - wie beschrieben - durch ein zweites Fluid gesteuert, welches den Kolben in das Reservoir drückt. Dadurch wird das im Reservoir vorliegende erste Fluid in das dilatierbare Element verdrängt.

Bei Rückführung des Kolbens wird das in das dilatierbare Element verdrängte Fluid durch Unterdruck wieder in das Reservoir zurückgeführt, wodurch das dilatierbare Element wieder in seinen nicht-dilatierbaren Zustand überführt wird. Dadurch kann einerseits eine schnelle und effiziente Unterbrechung des Blutstroms und andererseits eine zuverlässige Freigabe des Hohlkörpers erzielt werden.

In einer anderen Ausführungsform ist bevorzugt, wenn die im Reservoir vorgesehenen Mittel ein zweites dilatierbares Element darstellen, welches im Reservoir durch Zuleitung des zweiten Fluids in das zweite dilatierbare Element von einem nicht-dilatierten Zustand in einen dilatierten Zustand überführbar ist.

Bei dieser Ausführungsform wird also das zweite Fluid in das im Reservoir vorgesehene zweite dilatierbare Element eingeleitet. Dadurch wird das Element dilatiert und verdrängt mit seinem Volumen das im Reservoir vorliegende erste Fluid. Das erste Fluid, das somit aus dem Reservoir verdrängt wird, wird über die Öffnung des ersten dilatierbaren Elements, das über diese Öffnung mit dem Reservoir in Fluidverbindung steht, in das erste dilatierbare Element gedrückt, wodurch dieses sich erweitert und den Hohlkörper verschließt. Auch diese Ausführungsform hat den Vorteil, dass eine schnelle Unterbrechung des Blutstroms durch den Hohlkörper erzielt werden kann, wodurch vorteilhaft das Weiterführen von Gas- oder Feststoffpartikeln im Blutstrom vermieden wird.

Das im Reservoir vorgesehene zweite dilatierbare Element ist dabei an einer Wand des Reservoirs derart angebracht, dass es mit seinem dilatierbaren Teil in das Reservoir hineinragt und dieses gleichzeitig dichtend abschließt. Ferner weist das zweite dilatierbare Element eine Öffnung auf, über welche es mit dem Raum außerhalb des Reservoirs, insbesondere mit einer Zufuhr für das zweite Fluid, in Fluidverbindung steht. Auch bei dieser Ausführungsform ist also vorteilhaft gewährleistet, dass das im Reservoir vorliegende erste Fluid nicht über undichte Stellen entweichen kann. Auch bei dieser Ausführungsform ist also vorteilhaft gewährleistet, dass das im Reservoir vorliegende erste Fluid zuverlässig in das erste dilatierbare Element überführt wird.

In einer weiteren Ausführungsform ist bevorzugt, wenn die im Reservoir vorgesehenen Mittel eine Membran darstellen, welche im Reservoir durch Zuleitung des zweiten Fluids von einer ersten Position in eine zweite Position überführbar ist.

Bei dieser Ausführungsform befindet sich also eine Membran im Reservoir, die das Reservoir bspw. in seiner gesamten Länge durchspannen kann und es dadurch einen ersten Raum teilt, der über die Öffnung direkt mit dem dilatierbaren Element in Fluidverbindung steht, und in einen zweiten Raum, der mit dem zweiten Fluid in Fluidverbindung steht. Die Membran befindet sich in einer ersten Position, wenn das dilatierbare Element sich im nicht-dilatierten Zustand befindet. Dabei befindet sich in dem Raum des Reservoirs, welcher mit dem dilatierbaren Element in direkter Fluidverbindung steht, eine bestimmte Menge des ersten Fluids. Aufgrund der direkten Fluidverbindung des Reservoirs mit dem dilatierbaren Element kann dabei das erste Fluid auch teilweise in dem dilatierbaren Element vorliegen, ohne dieses jedoch in einen expandierten Zustand zu überführen, wenn sich die Membran in der ersten Position befindet.

Wird das zweite Fluid in den Raum des Reservoirs eingeleitet, welcher mit dem zweiten Fluid in Fluidverbindung steht, wird Druck auf die Membran ausgeübt. Dadurch wird die Membran von einer ersten Position in eine zweite Position überführt, wodurch die Membran in den ersten Raum des Reservoirs bewegt wird, in welchem das erste Fluid vorliegt. Dadurch wird das erste Fluid aus dem Reservoir verdrängt, und wird über die Öffnung des ersten dilatierbaren Elements, das über diese Öffnung mit dem Reservoir in Fluidverbindung steht, in das erste dilatierbare Element gedrückt, wodurch dieses sich erweitert und den Hohlkörper verschließt.

Auch diese Ausführungsform hat den Vorteil, dass eine schnelle Unterbrechung des Blutstroms durch den Hohlkörper erzielt werden kann, wodurch vorteilhaft das Weiterführen von Gas- oder Feststoffpartikeln im Blutstrom vermieden wird.

Vorliegend bedeutet "Membran" jedes folien-, schicht- oder blattartig ausgebildete Element, das zum einen elastische Eigenschaften aufweist, und andererseits hinreichend Spannkraft aufweist, um einem bestimmten, durch ein Fluid erzeugten Druck entgegenzuwirken bzw. diesem nachzugeben. Die Membran kann dabei ein ähnliches Material aufweisen oder aus diesem bestehen wie das zweite dilatierbare Element in der oben beschriebenen Ausführungsform. Insbesondere ist bevorzugt, wenn es sich hierbei um ein Wasserdampf-dichtes oder nahezu Wasserdampf-dichtes Material handelt.

Insgesamt ist bei der erfindungsgemäßen Vorrichtung ferner bevorzugt, wenn ein Ventil vorgesehen ist, mit welchem die Zuleitung des zweiten Fluids regelbar ist.

Es ist außerdem bevorzugt, wenn das erste im Reservoir vorgesehene Fluid aus einer Reihe hochviskoser Flüssigkeiten oder Gase ausgewählt ist, bspw. aus der Reihe der Infusionslösungen, physiologische Kochsalzlösungen, Blutexpander, künstliches Blut etc.

Ferner ist bevorzugt, wenn das zweite, die im Reservoir vorgesehenen Mittel steuernde Fluid aus der Reihe der Gase bzw. deren Gemische - bspw. atmosphärische Luft - ausgewählt ist.

Dies hat den Vorteil, dass - sollte es wider Erwarten zu einer Leckage des ersten dilatierbaren Elements kommen - gewährleistet ist, dass es bei Anwendung im extrakorporalen Blutkreislauf zu keinem Übertritt von schädlichen oder toxischen Stoffen kommt.

Es ist ferner bevorzugt, wenn das erste dilatierbare Element bei Einsatz in einem extrakorporalen Kreislauf ein hämokompatibles Material aufweist.

Dadurch wird eine Reaktion des im Hohlkörper geführten Blutes durch Kontakt mit dem Material vermieden, was bei nicht-hämokompatiblen Materialien bspw. zu einer Komplementaktivierung führen kann.

Bei der erfindungsgemäßen Vorrichtung kann die Unterbrechung des Fluidstromes dadurch aufgehoben werden, dass der zweite Ballon entlüftet bzw. der im Reservoir geführte Kolben rückgeführt wird. Dadurch wird erreicht, dass das erste Fluid aus dem durch das Fluid dilatierten ersten Element über die Öffnung in das Reservoir zurückfließt. Auf diese Weise wird - wie oben beschrieben - das zumindest eine dilatierbare Element wieder in einen nicht-expandierbaren Zustand überführt und der Weg durch den Hohlkörper freigegeben. Dadurch bleibt die erfindungsgemäße Vorrichtung mehrfach einsetzbar.

Die Erfindung betrifft ferner ein Verfahren, mit welchem der Blutstrom in einem extrakorporalen Kreislauf unterbrochen werden kann, wobei die erfindungsgemäße Vorrichtung eingesetzt wird.

Die erfindungsgemäße Vorrichtung kann ferner zur Unterbrechung eines Blutstroms in einem extrakorporalen Kreislauf, insbesondere in einer extrakorporalen Lungenunterstützung mit einem Lung-Assist-Device verwendet werden.

Weitere Vorteile ergeben sich aus der Beschreibung und den beigefügten Zeichnungen.

Ausführungsbeispiele der Erfindung sind in der Zeichnung dargestellt und werden nachstehend anhand der Beschreibung näher erläutert. Es zeigen:
Fig. 1a einen Längsschnitt durch die erfindungsgemäße Vorrichtung mit einem dilatierbaren Ballon als dilatierbares erstes Element und einem weiteren im Reservoir vorgesehenen dilatierbaren Ballon, wobei sich beide Ballone im nicht-dilatierten Zustand befinden;
Fig. 1b die Vorrichtung aus Fig. 1a, jedoch diesmal mit beiden Ballonen im dilatierten Zustand;
Fig. 2a eine weitere Ausführungsform der erfindungsgemäßen Vorrichtung im Längsschnitt, wobei das zumindest eine erste dilatierbare Element ein Ballon ist und wobei ferner ein Kolben vorgesehen ist, der im Reservoir verschiebbar angebracht ist;
Fig. 2b die Vorrichtung aus Fig. 2a, wobei diesmal der Ballon durch den eingeführten Kolben im expandierten Zustand ist;
Fig. 3a eine weitere Ausführungsform der erfindungsgemäßen Vorrichtung, wobei das zumindest eine erste dilatierbare Element ein entlang des Hohlkörperquerschnitts geführter dilatierbarer Schlauch ist, und im Reservoir ein weiterer dilatierbarer Ballon vorgesehen ist, wobei sich jedoch beide Elemente im nicht-dilatierten Zustand befinden;
Fig. 3b die Vorrichtung aus Fig. 3a, wobei jedoch beide dilatierbaren Elemente expandiert sind;
Fig. 4a noch eine weitere Ausführungsform der erfindungsgemäßen Vorrichtung, wobei das dilatierbare Element wie in Fig. 3a und 3b ein an der Hohlkörperinnenseite entlang des Querschnitts geführter Schlauch ist und wobei wie in Fig. 2a und 2b ein Kolben im Reservoir verschiebbar angebracht ist;
Fig. 4b die Vorrichtung aus Fig. 4a, wobei der innere Schlauch durch Einführen des Kolbens in das Reservoir und durch das dadurch verursachte Eindringen des ursprünglich im Reservoir vorliegenden Fluids in den inneren Schlauch im expandierten Zustand ist;
Fig. 5a eine weitere Ausführungsform der erfindungsgemäßen Vorrichtung, wobei das eine erste dilatierbare Element ein Ballon ist und wobei ferner eine bewegliche Membran im Reservoir vorgesehen ist, die sich in einer ersten Position befindet; und
Fig. 5b die Vorrichtung aus Fig. 5a, wobei durch Überführen der Membran im Reservoir von einer ersten Position in eine zweite Position das im Reservoir vorliegende Fluid den Ballon in einen expandierten Zustand überführt.

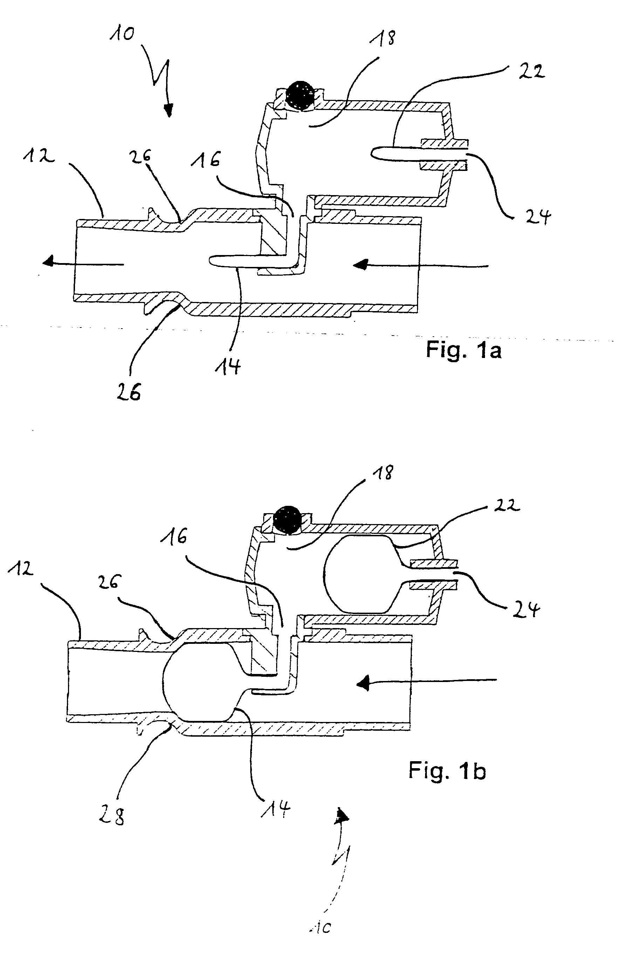

In Fig. 1a bezeichnet 10 insgesamt eine Vorrichtung zur Unterbrechung eines durch einen Hohlkörper 12 fließenden Blutstroms. Die Vorrichtung 10 weist ein erstes dilatierbares Element 14 in Form eines Ballons auf, das sich in Fig. 1a im nicht-dilatierten Zustand befindet. Das Element weist eine Öffnung 16 auf, über die es mit einem Reservoir 18 in Fluidverbindung steht. Im Reservoir 18 befindet sich das erste Fluid, mit welchem das dilatierbare Element 14 in einen expandierten Zustand überführt werden kann. Die Vorrichtung 10 weist ferner ein zweites dilatierbares Element 22 auf, welches in das Reservoir 18 hineinragt, wobei sich in Fig. 1a dieses Element 22 im nicht-dilatierten Zustand befindet. Das zweite dilatierbare Element 22 weist eine Öffnung 24 auf, über die es mit einem zweiten Fluid dilatiert werden kann.

In der Vorrichtung 10 in Fig. 1a ist ferner mit dem Bezugszeichen 26 eine ringförmige (Querschnitts-)Verengung im Hohlkörper 12 gekennzeichnet, an welcher sich das dilatierte Element 14 dichtend anlegt.

In Fig. 1b befindet sich das dilatierbare Element 14 im dilatierten Zustand, ebenso wie das dilatierbare Element 22 im Reservoir 18.

Die Funktionsweise der erfindungsgemäßen Vorrichtung ist derart, dass wenn der Blutstrom unterbrochen werden soll - bspw. aufgrund eines Gaseintritts - das dilatierbare Element 22 durch Einleitung eines zweiten Fluids durch die Öffnung 24 in einen dilatierten Zustand gebracht wird. Durch die Volumenvergrößerung des dilatierbaren Elements 22 wird das im Reservoir 18 vorliegende Fluid durch die Öffnung 16 in das dilatierbare Element 14 verdrängt. Durch das eindringende Fluid wird das dilatierbare Element 14 in einen dilatierten Zustand gebracht, in welchem das dilatierbare Element 14 dichtend an dem ringförmigen Vorsprung 26 anschließt und dadurch den Hohlkörper 12 verschließt. Der Fluidstrom, bzw. dessen Unterbrechung, ist in Fig. 1a und 1b in der Vorrichtung 10 mit Pfeilen angedeutet.

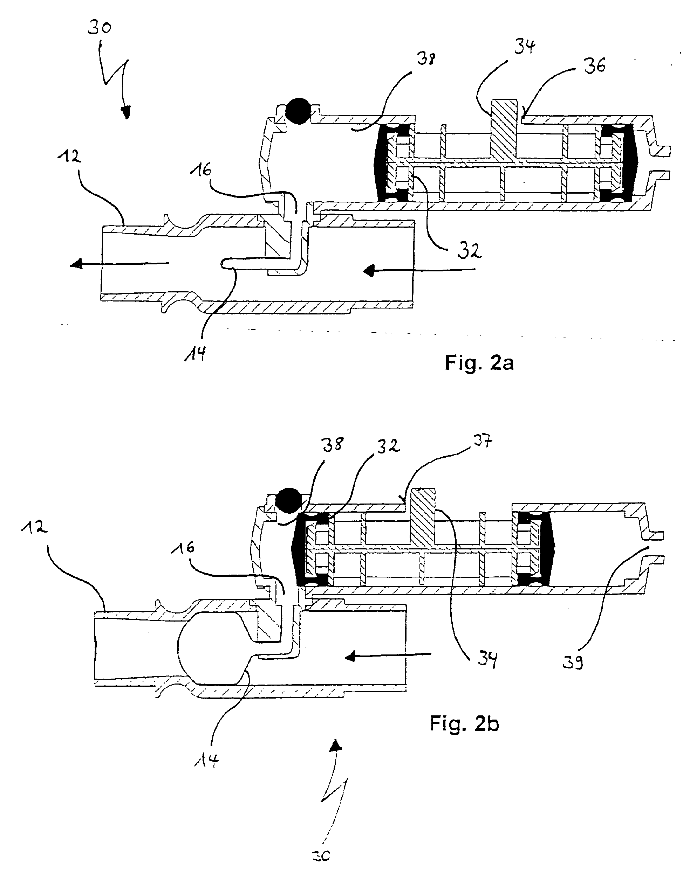

In Fig. 2a ist eine andere Ausführungsform der erfindungsgemäßen Vorrichtung 30 gezeigt, wobei diejenigen Elemente, die diese Ausführungsform mit der Ausführungsform aus Fig. 1a und 1b gemeinsam hat, mit den gleichen Bezugszeichen versehen sind. Auch diese Ausführungsform weist ein im Hohlkörper 12 vorliegendes dilatierbares Element 14 mit einer Öffnung 16 auf, über die das dilatierbare Element 14 mit einem Reservoir 38 in Fluidverbindung steht. Das dilatierbare Element 14 befindet sich in Strömungsrichtung im Hohlkörper 12, und zwar in einem nicht-dilatierten Zustand (Fig. 2a). Im Reservoir 38 ist ein Kolben 32 verschiebbar angebracht. Der Kolben 32 weist einen in einer Nut geführten Stift 34 auf, über welchen sich die Auslenkung des Kolbens 32 im Reservoir 38 begrenzen lässt. In Fig. 2a ist eine erste Position des Kolbens 32 gezeigt, in welchem der Großteil des Reservoirs 38 freigegeben ist, wodurch im Reservoir 38 Raum für das Fluid ist. Die Auslenkung des Kolbens 32 aus dem Reservoir 38 hinaus wird durch den Stift 34 begrenzt, welcher an einer Stelle 36 des Reservoirs 38 an dessen Wandung stößt und dadurch den Lauf des Kolbens 32 aus dem Reservoir 38 stoppt.

In Fig. 2b ist gezeigt, dass der Kolben 32 im Reservoir 38 in eine zweite Position verschoben wurde, wodurch das im Reservoir 38 vorliegende Fluid über die Öffnung 16 in das dilatierbare Element 14 verdrängt wurde. Dadurch wird das dilatierbare Element 14 in einen expandierten Zustand überführt und schließt an der Stelle 26 an der ringförmigen Verengung im Hohlkörpersystem 12 dichtend ab, wodurch der Blutstrom im Hohlkörpersystem 12 unterbrochen wird. Die Führung des Kolbens 32 in das Reservoir 38 hinein wird dabei durch das Anstoßen des Stifts 34 an eine zweite Stelle 37 der Wandung des Reservoirs 38 begrenzt.

Der Kolben 32, bzw. dessen Führung, wird dabei über ein zweites Fluid gesteuert, das über die Öffnung 39 zu- und abgeführt wird.

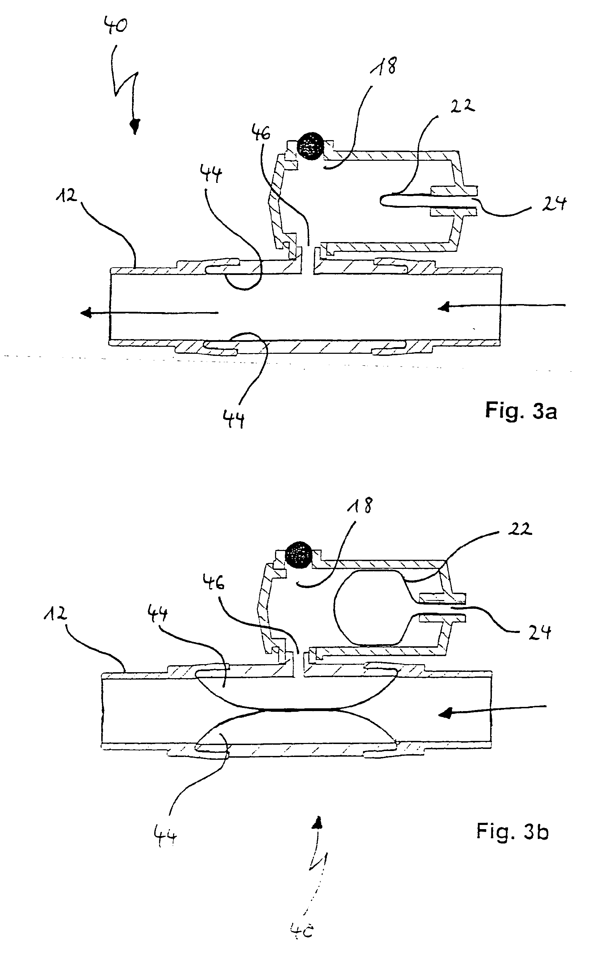

In Fig. 3a ist eine andere Ausführungsform der erfindungsgemäßen Vorrichtung 40 gezeigt, wobei auch hier gleiche Elemente wie in den Figuren 1 und 2 mit den gleichen Bezugszeichen versehen sind. Die Vorrichtung 40 weist wiederum ein Reservoir 18 sowie im Reservoir 18 ein dilatierbares Element 22 mit einer Öffnung 24 auf, über die das zweite Fluid zugeführt werden kann. Die Vorrichtung 40 weist ferner ein an der Innenwand des Hohlkörpers 12 geführtes dilatierbares Element 44 in Form eines Schlauches auf, das sich in Fig. 3a im nicht-dilatierten Zustand an die innere Oberfläche des Hohlkörpers 12 entlang dessen Querschnitt anschmiegt. Das dilatierbare Element 44 ist über eine Öffnung 46 mit dem Reservoir 18 verbunden ist.

Wird nun das zweite dilatierbare Element 22 durch Zuleitung eines Fluids über die Öffnung 24 in einen expandierten Zustand gebracht, wird das im Reservoir 18 vorliegende Fluid über die Öffnung 46 in das erste dilatierbare Element 44 verdrängt, wodurch dieses wiederum in einen dilatierten Zustand überführt wird. Das dilatierbare Element 44, das in Fig. 3 als Schlauch vorliegt, verschließt dabei in dilatierten Zustand den Hohlkörper 12. Diese Position ist in Fig. 3b gezeigt.

Durch Abführen des zweiten Fluids aus dem dilatierbaren Element 22 im Reservoir 18 wird das dilatierbare Element 22 in einen nicht-dilatierten Zustand überführt. Durch diese Volumenreduzierung wird das erste Fluid durch Unterdruck in das Reservoir 18 zurückgeführt, wodurch das dilatierbare Element 44 wieder in den nicht-dilatierten Zustand überführt wird.

In Fig. 4a ist eine weitere Ausführungsform der erfindungsgemäßen Vorrichtung 50 gezeigt, die wie in Fig. 3 ein an der Innenwand des Hohlkörpers 12 geführtes dilatierbares Element 44 in Form eines Schlauches aufweist, das in Fig. 4a im nicht-dilatierten Zustand gezeigt ist. Das dilatierbare Element 44 in Fig. 4a schmiegt sich im nicht-dilatierten Zustand an die innere Oberfläche des Hohlkörpers 12 entlang dessen Querschnitt an. Das dilatierbare Element 44 steht über die Öffnung 46 mit dem Rservoir 38 in Fluidverbindung.

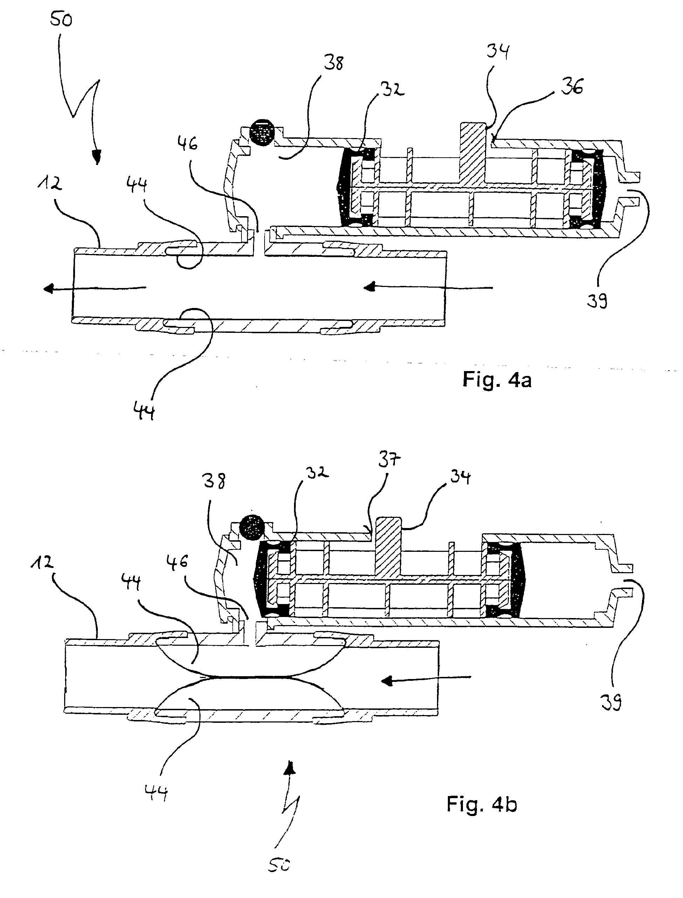

In dem Reservoir 38 in Fig. 4 ist ein Kolben 32 vorgesehen, der im Reservoir 38 verschiebbar angebracht ist. Wie in Fig. 2 weist der Kolben 32 einen in einer Nut geführten Stift 34 auf, über welchen sich die Auslenkung des Kolbens 32 im Reservoir 38 begrenzen lässt. In Fig. 4a ist eine erste Position des Kolbens 32 gezeigt, in welchem der Großteil des Reservoirs 38 freigegeben ist, wodurch im Reservoir 38 Raum für das Fluid ist. Die Auslenkung des Kolbens 32 aus dem Reservoir 38 hinaus wird durch den Stift 34 begrenzt, welcher an einer Stelle 36 des Reservoirs 38 an dessen Wandung stößt und dadurch den Lauf des Kolbens 32 aus dem Reservoir 38 stoppt.

In Fig. 4b ist wie in Fig. 2b gezeigt, dass der Kolben 32 im Reservoir 38 in eine zweite Position verschoben werden kann, wodurch das im Reservoir 38 vorliegende Fluid über die Öffnung 46 in das dilatierbare Element 44 verdrängt wird. Dadurch wird das dilatierbare Element 44 in einen expandierten Zustand überführt und schließt an der Stelle 26 an der ringförmigen Verengung des Hohlkörpers 12 dichtend ab, wodurch der Blutstrom im Hohlkörper 12 unterbrochen wird. Die Führung des Kolbens 32 in das Reservoir 38 hinein wird dabei durch das Anstoßen des Stifts 34 an eine zweite Stelle 37 der Wandung des Reservoirs 38 begrenzt.

Der Kolben 32, bzw. dessen Führung, wird dabei wie in Fig. 2 über ein zweites Fluid gesteuert, das über die Öffnung 39 zu- und abgeführt wird.

Durch Rückstellen des Kolbens 32 in die Ausgangsposition wird das erste Fluid durch den entstehenden Unterdruck aus dem dilatierbaren Element 44 über die Öffnung 46 in das Reservoir 38 zurückgeführt.

In Fig. 5a ist eine weitere Ausführungsform der erfindungsgemäßen Vorrichtung 60 gezeigt, wobei diejenigen Elemente, die diese Ausführungsform mit der Ausführungsform aus Fig. 1a, 1b, 2a und 2b gemeinsam hat, mit den gleichen Bezugszeichen versehen sind.

In Fig. 5a bezeichnet 60 insgesamt eine Vorrichtung zur Unterbrechung eines durch einen Hohlkörper 12 fließenden Blutstroms. Die Vorrichtung 60 weist ein erstes dilatierbares Element 14 in Form eines Ballons auf, das sich in Fig. 1a im nicht-dilatierten Zustand befindet. Das Element weist eine Öffnung 16 auf, über die es mit einem Reservoir 18 in Fluidverbindung steht. Durch das Reservoir erstreckt sich eine Membran 62, die das Reservoir 18 dadurch räumlich in einen ersten Teil 64 und einen zweiten Teil 65 aufteilt. Der erste Teil 64 steht über die Öffnung 16 direkt mit dem dilatierbaren Element 14 in Fluidverbindung. Der zweite Teil 65 steht mit dem zweiten Fluid in Fluidverbindung.

Wie in Fig. 5a befindet sich die Membran 62 in einer ersten Position, wenn das dilatierbare Element 14 sich im nicht-dilatierten Zustand befindet. In Fig. 5a ist dies durch eine leichte Wölbung der Membran 62 in Richtung des zweiten Teils des Reservoirs gezeigt. In dem ersten Teil befindet sich eine bestimmte Menge des ersten Fluids. Zum Teil kann sich das erste Fluid in geringen Mengen auch im dilatierbaren Element 14 befinden, ohne dieses jedoch in eine dilatierten Zustand zu versetzen.

In der Vorrichtung 60 in Fig. 5a ist wiederum mit dem Bezugszeichen 26 eine ringförmige (Querschnitts-)Verengung im Hohlkörper 12 gekennzeichnet, an welcher sich das dilatierte Element 14 dichtend anlegt.

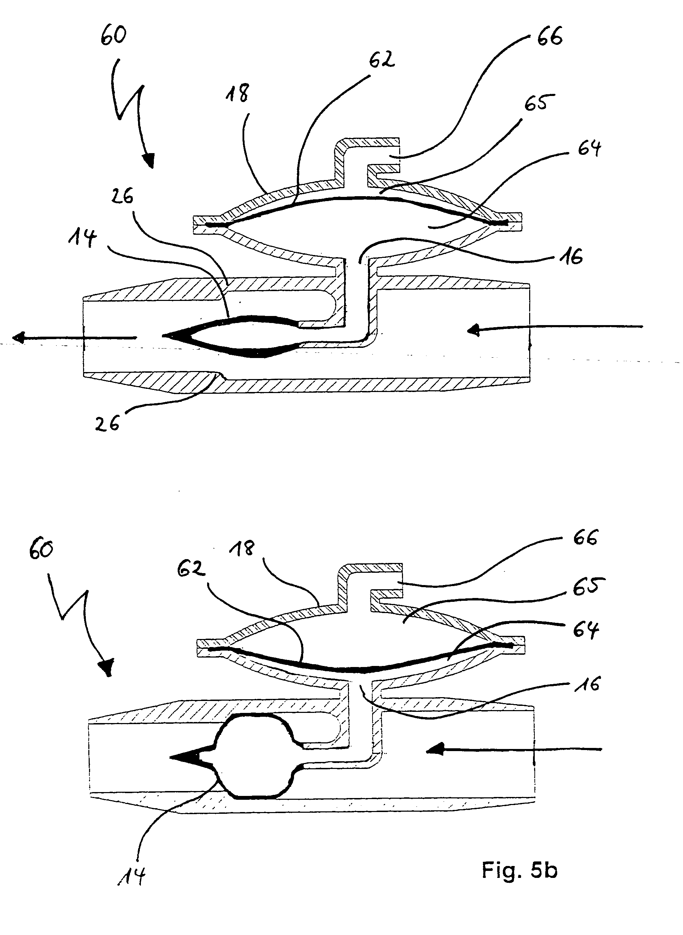

In Fig. 5b befindet sich das dilatierbare Element 14 im dilatierten Zustand.

Die Funktionsweise der erfindungsgemäßen Vorrichtung 60 ist derart, dass wenn der Blutstrom unterbrochen werden soll - bspw. aufgrund eines Gaseintritts - über die Öffnung 66 ein zweites Fluid in das Reservoir 18, bzw. in den zweiten Teil 65 des Reservoirs 18 zugeleitet wird. Durch das zweite Fluid wird Druck auf die Membran 62 ausgeübt, die diesen Druck wiederum aufgrund Ihrer Elastizität auf das im ersten Teil 64 vorliegende erste Fluid weitergibt. Dadurch wird dieses wiederum aus dem ersten Teil 64 des Reservoirs 18 über die Öffnung 16 in das dilatierbare Element 14 gedrückt, wodurch dieses sich dilatiert. Das dilatierbare Element 14 schließt dichtend an dem umlaufenden ringförmigen Vorsprung 26 an und verschließt dadurch den Hohlkörper 12. Der Fluidstrom, bzw. dessen Unterbrechung, ist in den Fig. 5a und 5b in der Vorrichtung 60 mit Pfeilen angedeutet.
In Fig. 5b ist gezeigt, dass sich die Membran nach Einleiten des zweiten Fluids in den zweiten Teil 65 des Reservoirs 18 in den ersten Teil 64 wölbt, wodurch das Fluidvolumen im ersten Teil 64 des Reservoirs 18 wie erwähnt in das dilatierbare Element 14 verdrängt wird.

Durch Abführen das zweiten Fluids aus dem Reservoir 18, bzw. dem zweiten Teil 65 des Reservoirs 18, wird die Membran "entlastet" und in ihre erste Position zurückgeführt. Dadurch wird das erste Fluid durch Unterdruck aus dem dilatierbaren Element 14 geführt, wodurch dieses wieder in einen nicht-dilatierten Zustand gebracht wird.

## Patentansprüche

1. Vorrichtung zur Unterbrechung eines durch einen Hohlkörper fließenden Blutstroms in einem extrakorporalen Blutkreislauf, mit einem röhrenförmigen Hohlkörper (12) und mit zumindest einem in dem Hohlkörper (12) angeordneten dilatierbaren Element (14; 44), welches eine Öffnung (16; 46) aufweist, über welche das zumindest eine dilatierbare Element (14; 44) mit einem Fluidanschluss in Fluidverbindung steht, wobei das zumindest eine dilatierbare Element (14; 44) durch Zuleitung eines Fluids aus dem Fluidanschluss durch die Öffnung (16; 46) von einem nicht-dilatierten in einen dilatierten Zustand überführbar ist, wobei das zumindest eine dilatierbare Element (14; 44) im dilatierten Zustand den Blutstrom unterbricht, **dadurch gekennzeichnet, dass** ferner Mittel (22; 32; 62) im Fluidanschluss vorgesehen sind, welche die Zuleitung des Fluids in das zumindest eine dilatierbare Element (14; 44) steuern, wobei die Mittel (22; 32; 62) über ein zweites Fluid steuerbar sind.

2. Vorrichtung nach Anspruch 1, **dadurch gekennzeichnet, dass** der Fluidanschluss ein Reservoir (18; 38) ist.

3. Vorrichtung nach Anspruch 2, **dadurch gekennzeichnet, dass** die Volumenmenge an Fluid, die in dem Reservoir (18; 38) enthalten ist, zumindest der Volumenmenge des zumindest einen dilatierbaren Elements (14; 44) im expandierten Zustand entspricht.

4. Vorrichtung nach einem der Ansprüche 1 bis 3, **dadurch gekennzeichnet, dass** das zumindest eine dilatierbare Element (14) ein Ballon ist.

5. Vorrichtung nach einem der Ansprüche 1 bis 4, **dadurch gekennzeichnet, dass** das Mittel ein Kolben (32) ist, der über das zweite Fluid verschiebbar im Reservoir (38) angebracht ist.

6. Vorrichtung nach einem der Ansprüche 1 bis 4, **dadurch gekennzeichnet, dass** das Mittel ein zweites dilatierbares Element (22) ist, welches im Reservoir (18) durch Zuleitung des zweiten Fluids in das zweite dilatierbare Element (22) von einem nicht-dilatierten Zustand in einen dilatierten Zustand überführbar ist.

7. Vorrichtung nach einem der Ansprüche 1 bis 4, **dadurch gekennzeichnet, dass** das Mittel eine Membran (62) ist, welche im Reservoir durch Zuleitung des zweiten Fluids von einer ersten Position in eine zweite Position überführbar ist.

8. Vorrichtung nach einem der Ansprüche 1 bis 7, **dadurch gekennzeichnet, dass** ferner ein Ventil vorgesehen ist, mit welchem die Zuleitung des zweiten Fluids regelbar ist.

9. Vorrichtung nach einem der Ansprüche 1 bis 8, **dadurch gekennzeichnet, dass** das im Reservoir enthaltende Fluid aus der Gruppe hochviskoser physiologischer Flüssigkeiten oder Gase ausgewählt ist, wie bspw. Blutexpander, künstliches Blut.

10. Vorrichtung nach einem der Ansprüche 1 bis 9, **dadurch gekennzeichnet, dass** das eine dilatierbare Element (14) bei Einsatz im extrakorporalen Kreislauf ein hämokompatibles Material aufweist.

11. Verfahren zur Unterbrechung eines Blutstromes, **dadurch gekennzeichnet, dass** eine Vorrichtung nach einem der Ansprüche 1 bis 10 eingesetzt wird.

12. Verwendung einer Vorrichtung nach einem der Ansprüche 1 bis 10 zur Unterbrechung eines Blutstromes in einem extrakorporalen Kreislauf.

13. Verwendung einer Vorrichtung nach einem der Ansprüche 1 bis 10 in einer extrakorporalen Lungenunterstützung mit einem Lung-Assist-Device.

14. Verwendung eines über ein Fluid dilatierbaren Elements in einem extrakorporalen Blutkreislauf zur Unterbrechung eines durch einen Hohlkörper fließenden Blutstroms beim Auftreten von Gasbläschen oder Tromben.
